(19) Europäisches Patentamt / European Patent Office / Office européen des brevets

(11) **EP 4 765 153 A1**

(12) **EUROPEAN PATENT APPLICATION**

(43) Date of publication:
24.06.2026   Bulletin 2026/26

(21) Application number: 24222782.5

(22) Date of filing: **23.12.2024**

(51) International Patent Classification (IPC):
***G16H 40/20*** (2018.01)

(52) Cooperative Patent Classification (CPC):
**G16H 40/20**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Siemens Healthineers AG
91301 Forchheim (DE)**

(72) Inventors:
• **ROUT, Pratyush
  Bangalore, Karnataka 560100 (IN)**
• **SUNDARAPANDIAN, Manivannan
  Bangalore, 560030 (IN)**
• **BEILNER, Janina
  81675 München (DE)**

(74) Representative: **HKW Intellectual Property PartG
mbB
Theresienhöhe 12
80339 München (DE)**

(54) **SYSTEM AND METHOD FOR ALLOCATING MEDICAL STAFF TO A NUMBER OF MEDICAL DEVICES**

(57)   The invention relates to a system for allocating medical staff to medical devices, a method and a computer program product.

A system for allocating medical staff to a number N1 of medical devices, with $N1 \geq 1$, and for controlling the N1 medical devices, the system comprising: a user interface for acquiring input data and adding the acquired input data to a set of constraints; a scheduler unit for providing a number N2 of schedules by allocating a number N3 of allocated medical staff out of a number N4 of total medical staff to the N1 medical devices based on the set of constraints, with $N2 \geq 1$, $N3 \geq 1$, and $N4 \geq 1$; and the N1 medical devices; wherein the system is configured to control the N1 medical devices based on the N2 provided schedules.

The system can provide the allocation in a brief time while respecting multiple constraints, enabling regular updates. Eventually, the N1 medical devices can be operated in a more efficient manner.

FIG 1

EP 4 765 153 A1

**Description**

The present invention relates to a system for allocating medical staff to a number of medical devices, a method, and a computer program product.

In medical facilities, for example, in medium to large hospitals, using medical devices such as diagnostic systems or surgeon systems controlling and scheduling the medical devices as well as scheduling the medical staff using the medical devices is a challenging problem, that is, generating a usage scheme or schedule defining when, where, in which manner and by whom of the medical staff each of the medical devices of the medical facility will be used in the future. Said provides a workflow instruction for the medical facility, eventually influencing the manner how and when the medical devices will be used. In particular, in case scheduling reaches across various working shifts within the medical facility and/or across various departments of the medical facility, said scheduling is a challenging problem. Therefore, optimal controlling and scheduling of the medical staff and the medical devices is a key topic of interest in healthcare management, as it directly impacts the operational efficiency of a hospital. Independent of the grammatical term usage, individuals with male, female or other gender identities are included within the term.

Usually, the scheduling is done by a unit manager for a single planning horizon, covering for example the next four weeks. It is known to the applicant that for solving said scheduling, an automated scheduling software can be used in order to ease achieving a transparent and unbiased solution. However, as the number of the medical staff members, the number of medical devices, and/or the number of working shifts scale up, the problem can become impossible or time-consuming to solve in a satisfying manner, that is, with respect to possible given constraints. With the classical computing hardware, depending on problem complexity, the automated scheduling software can take several tens of minutes or more to generate a schedule for a single planning horizon. Furthermore, within automated scheduling software, the underlying model to generate the schedule cannot be tuned directly by the unit manager in order to minimize possible constraint violations. Hence, typically, the unit manager has to employ trial and error methods to tune the model which is inefficient and time-consuming and can cause an inadequate usage of the medical devices and/or an inadequate treatment of the patients. In addition, according to knowledge of the applicant, it is beneficial to allocate the medical staff to the medical devices in a balanced manner to achieve the best efficiency of the medical staff and/or the medical devices. That is, the members of the medical staff have to be allocated across various medical devices according to their individual different knowledge levels and experience with respect to every medical device. Hereby, the members of the medical device gain experience in using every medical device in a balanced manner. This can provide a safe, adequate, and efficient usage of the medical devices and an adequate treatment of the patients. Nonetheless, nowadays automated scheduling software still miss such a balanced allocation.

Therefore, it is one object of the present invention to improve allocating the medical staff to the number of medical devices.

Accordingly, a system for allocating medical staff to a number N1 of medical devices, with $N1 \geq 1$, and for controlling the N1 medical devices is suggested. The system comprises: a user interface for acquiring input data and adding the acquired input data to a set of constraints; a scheduler unit for providing a number N2 of schedules by allocating a number N3 of allocated medical staff out of a number N4 of total medical staff to the N1 medical devices and/or to a working shift based on the set of constraints, with $N2 \geq 1$, $N3 \geq 1$, and $N4 \geq 1$; and the N1 medical devices. Hereby, the system is configured to control the N1 medical devices based on the N2 provided schedules.

The system can be configured to allocate the medical staff to the N1 medical devices in an automatic manner. In other words, after acquiring the input data, the scheduler unit can be configured to provide the N2 schedules without or with a minimized need for user interactions. The N2 schedules may comprise instructions concerning when, by whom of the medical staff and in which manner each of the N1 medical devices is used or will be used in the future. Hereby, the N2 schedules may be based on specific values of a number of variables which are limited by the set of constraints.

The N1 medical devices may comprise any system, machine, device, or tool used for medical purposes. The N1 medical devices can work autonomously and/or can be used by members of the medical staff. For example, the N1 medical devices may comprise medical imaging devices such as CT scanners, MRT scanners, MRI scanners, invivo-diagnostic machines, X-ray devices, or the like. Hereby, for a given type of medical device, the N1 medical devices may further comprise different models of the given type.

The user interface can be realized as a computer and/or as a computer terminal. The user interface can comprise a desktop, a monitor, a touch screen, a keyboard, a mouse, and/or any other input or control device. Using the user interface, the user may communicate with the system, and both insert input data into the system as well as receive output data from the system. Hereby, the input data may comprise any data required to provide the N2 schedules, for example, information concerning the N4 total medical staff, such as the number N4, that is, how many members of the medical staff are available in total, the number of staff members being required within each working shift of the medical facility, the respective experience level of each member of the N4 total medical staff regarding each of the N1 medical devices, the planned working hours and days off of the medical staff members, etc. The input data may further comprise information regarding the N1 medical devices, for example, a list of all N1 medical devices, their availability in total, within specific working shifts, and/or for each day or week within each planning horizon covered by the N2 schedules. The input data may further comprise the required number and/or the required experience level of medical staff members that is required in order to operate a respective one of the N1 medical devices. In addition, the input data may further comprise additional constraints such as pending medical device malfunctions or the like.

The acquired input data may be added to a set of constraints. It is further feasible that the set of constraints may comprise internal data of the system, for example, data of previously provided schedules and/or information regarding the N1 medical devices. The set of constraints may represent entries of at least one database. The set of constraints may comprise all constraints required to provide the N2 schedules, hence, the set of constraints may represent all factors and/or limitations which need to be respected in order to generate the N2 schedules. Hereby, some of the constraints may have to be fulfilled completely while other constraints may have to be fulfilled as far as possible. For example, the constraints may comprise time slots when each of the N1 medical devices is available, the duration how long each of the N1 medical devices can be used in a row, the duration how long a specific usage of each of the N1 medical devices takes, the number of medical staff members being required for each of the N1 medical devices with respect to specific usages of the N1 medical devices, the weekdays and/or times a specific staff member should work, the number of days off of a specific staff member during one planning horizon, and/or others.

The scheduler unit may be a computer and/or a computer program product which is configured to provide the N2 schedules based on the set of constraints. Hereby, the scheduler unit may be configured to provide the N2 schedules in the frame of an iterative calculation process within which the scheduler unit is configured to aim on finding an optimal solution for respecting the set of constraints. The scheduler unit may comprise and/or may be configured to apply algorithms for providing the N2 schedules and/or respective machine learning tools. Each of the N2 schedules may cover one or more planning horizons. Hereby, each planning horizon may cover a future time window of one week, one month, one year, etc. Each of the N2 schedules may comprise instructions when and how each of the N1 medical devices is used within the respective planning horizon as well as by which one or more members of the N4 total medical staff.

The N4 total medical staff may represent all available staff members using the system, for example, all staff members of a medical department or of the whole medical facility. The N3 allocated medical staff may represent a subgroup of the N4 total medical staff. The N3 allocated medical staff may represent those of the N4 total medical staff who are allocated to the N1 medical devices in the frame of the provided N2 schedules. In other words, the N3 allocated medical staff may represent the subgroup of the N4 total medical staff which is selected to work due to the N2 schedules. Hereby, the N3 allocated medical staff may be allocated both regarding the N1 medical devices, meaning where each member of the N3 allocated medical staff will work according to the provided N2 schedules, as well as when to work at each of the N1 medical devices. Furthermore, the N3 allocated medical staff may be allocated across working shifts, meaning the provided N2 schedules define within which working shift each member of the N3 allocated medical staff will work as well as within which team and/or department of the medical facility. The working shifts may comprise, for example, a morning shift, an afternoon shift, and/or a night shift. It is further possible that the N3 allocated medical staff may be allocated according to additional dimensions with respect to their individual skill level, experience level, etc.

Controlling the N1 medical devices may comprise controlling the manner when, how, and by which staff member each of the N1 medical devices is used in the frame of the provided N2 schedules. Furthermore, controlling the N1 medical devices may comprise executing instructions concerning which operation or operations have to be performed by each medical device and in which manner. Hereby, the N1 medical devices may be controlled directly by the system, for example, by sending instructions and/or other data from the scheduler unit to the N1 medical devices. This way, the system interacts directly with the N1 medical devices and controls when and how they are operating. In addition, or alternatively, the system may be configured to provide instructions to the user regarding the controlling of the N1 medical devices. Said may be realized by visualizing the instructions on a monitor, a display, and/or the like and/or by printing the instructions using a printer, a plotter, and/or the like.

The system may enable the generation of the N2 schedules in brief time, for example within a time window of several seconds or minutes. This way, the N2 schedules may be updated regularly and/or when facing specific events like malfunctions and/or maintenance actions of the N1 medical devices, and/or illnesses and/or vacations of members of the N4 total medical staff. Furthermore, the system may be able to pay respect to a number of constraints when providing the N2 schedules. In addition, the system may allow the user to influence the generation of the N2 schedules in an easy and/or pinpointed manner, for example, by adapting the constraints. Eventually, the N1 medical devices may be operated in a more efficient manner and/or with a lower probability of malfunctions. For example, time slots with an idle state of one or more of the N1 medical devices caused by an unavailability of members of the medical staff and/or an inadequate usage of the N1 medical devices may be avoided.

According to an embodiment, the set of constraints comprises a number N5 of hard constraints, a number N6 of soft constraints, and/or weightings of the N6 soft constraints, with $N5 \geq 1$ and $N6 \geq 1$.

The N5 hard constraints are mandatory. That is, the N5 hard constraints have to be completely fulfilled in order to provide the N2 schedules. Any violation of one of the N5 hard constraints due to the N2 schedules prevents the scheduler unit from providing the N2 schedules to the user. In other words, the scheduler unit is configured to adjust the N2 schedules until each of the N5 hard constraints is fulfilled. The N5 hard constraints can comprise, for example, a given availability of the N1 medical devices, a given shift rotation of the N4 total medical staff, that is, for example, that after a staff member has been working within a specific working shift for one or more weeks, the respective staff member will work in another working shift in the subsequent week. The N5 hard constraints may further comprise other mandatory requirements, for example a certain number of staff members being required to work with a specific medical device at a given time and/or for a given operation of the specific medical device. In contrast, the N6 soft constraints are desirable but optional. In other words, an increased degree of fulfilling of the N6 soft constraints may improve the quality of the N2 schedules. Nonetheless, a possible violation of the N6 soft constraints still enables providing the N2 schedules. The N6 soft constraints can comprise, for example, the requirement to provide a specific number of days off per week for each staff member, a specific number of hours a given staff member should work within a specific week, etc. The weightings can be set by the user and/or the system and can define the importance of each of the N6 soft constraints. The higher the importance of a given one of the N6 soft constraints is, the more the scheduler unit aims on fulfilling the given soft constraint when providing the N2 schedules, for example, within the iterative calculation process. In other words, with a rising weighting, the fulfilling of the given soft constraint is more likely. Hereby, the weightings can define the importance of each of the N6 soft constraints in relation to the respective other ones of the N6 soft constraints. In addition, or alternatively, the weightings can define the importance of the N6 soft constraints as a whole in relation to the N5 hard constraints. The weightings can be updated while using the system. For example, the weighting of a given one of the N6 soft constraints can be updated according to a number of times the given soft constraint is selected for adjustment by the user. In addition, or alternatively, the weighting of the given soft constraint can be updated according to an extent of violation of a value and/or range of the given soft constraint which is defined as being acceptable by the user. Each one of the N6 soft constraints can be addressed with a specific weighting. The user is provided with an easy manner for influencing the N2 schedules by adjusting the weightings and/or the allowable ranges of the N6 soft constraints. The weightings can be stored within a local memory of the system and/or on a cloud server.

According to a further embodiment, the N6 soft constraints comprise a required allocation of the N4 total medical staff to the N1 medical devices, in particular, the weightings comprise a weighting of the required allocation in relation to the others of the N6 soft constraints.

The required allocation refers to the time window of the provided N2 schedules, in particular, to the time window of a consecutive next one of the N2 provided schedules. The required allocation represents such an allocation of the N4 total medical staff to the N1 medical devices which is desired in order to converge towards or to achieve a balanced allocation over time. Hereby, the balanced allocation can be understood such way that when achieving and/or when proceeding a balanced allocation, every one of the N4 total medical staff has the same level of experience concerning the usage of a specific one of the N1 medical devices. In other words, the allocation of the N4 total medical staff may change over time such way that every one of the N4 total medical staff gains experience in using every one of the N1 medical devices, in particular, simultaneously. In addition, or alternatively, the balanced allocation can be understood as such allocation, according to which staff members with higher levels of experience are assigned to work with staff members with lower levels of experience regarding a specific medical device in order to provide a perspective transfer of experience. Hence, when a given one of the N4 total medical staff has low experience in using a given one of the N1 medical devices, the required allocation of the provided N2 schedules comprises an increased allocation of the given staff member to the given medical device, for example, by an increased intensity and/or time duration of the usage of the given medical device by the given staff member. This way, the experience of the given staff member in using the given medical device is increased over time. Therefore, the higher N2 is, that is, the more planning horizons the provided N2 schedules cover, the more precisely the balanced allocation can be achieved. With the N6 soft constraints comprising the required allocation, the N2 schedules can be adapted according to the level of experience of each of the N4 total medical staff, providing a learning effect for each of the N4 total medical staff according to the current level of experience.

Eventually, with increasing levels of experience, an increased quality and efficiency of the usage of the N1 medical devices can be achieved.

According to a further embodiment, the scheduler unit is configured to provide the N2 schedules by: generating a first schedule of the N2 schedules based on the set of constraints, in particular by setting a predefined initial distribution for a machine distribution constraint, wherein the machine distribution constraint is one of the N6 soft constraints; displaying the generated first schedule to a user of the system; optionally improving the generated first schedule; and calculating an allocation error of each of the N6 soft constraints based on a deviation between a scheduled allocation of the first schedule and the required allocation. In particular, the scheduler unit is configured to provide the N2 schedules by adjusting the weightings for at least one further schedule of the N2 schedules and repeating steps above for the at least one further schedule.

Hereby, the first schedule can cover one planning horizon, for example one week or one month. The machine distribution constraint can be a quantitative and/or qualitative information concerning how each member of the N4 total medical staff was allocated to the N1 medical devices in the past. Hence, the machine distribution constraint can represent a skill level for each one of the N4 total medical staff regarding each one of the N1 medical devices. The machine distribution constraint can be a vector, an array, or a matrix. For generating the first schedule, the machine distribution constraint can be set to the predefined initial distribution. The predefined initial distribution can be, for example, to set a uniform distribution, thus, setting uniform values for all entries of the machine distribution constraint, representing a uniform skill level of each of the N4 total medical staff regarding each one of the N1 medical devices. By improving the generated first schedule, the user has the possibility to modify the generated first schedule if necessary. To improve the generated first schedule can mean to adjust the generated first schedule such way that the generated first schedule meets the set of constraints with fever violations and/or with lower degrees of violation. For example, the user can adjust the set of constraints, in particular, the N6 soft constraints, for example, by adjusting the allowable ranges of the N6 soft constraints and/or by adjusting the weightings of the N6 soft constraints, followed by a regeneration of the first schedule. Possible violations of the N6 soft constraints can cause deviations between the scheduled allocation of the first schedule and the required allocation. In other words, with the calculated first schedule, the balance allocation is not met precisely. Hereby, the scheduled allocation represents the allocation of the N3 allocated medical staff to the N1 medical devices as calculated by the scheduler unit for a given planning horizon and/or within the generated first schedule. The deviation of each of the N6 soft constraints results in the calculated allocation error which describes the degree of the respective deviation.

In the following, one example of a method for calculating the allocation error and adjusting the weightings is described. Let the N1 medical devices to be used within a given planning horizon and/or within the N2 schedules be [M1, M2, M3, ... ]. Furthermore, the number of staff members required in order to operate the N1 medical devices can be [t1, t2, t3, ...]. This way, the number, $R_{ik}$, of working slots to be assigned for each staff member, i, during which the staff member i is working with a medical device, k, can be given as $R_{ik}$ = [tl/N3, t2/N3, t3/N, ... ] with N3 as the number of allocated medical staff for the given planning horizon and/or the N2 schedules. This way, $R_{ik}$ describes a required allocation value for the $i^{th}$ staff member in the $k^{th}$ medical device and represents the ideal case. In other words, with $R_{ik}$, a balanced allocation can be achieved. Initially, before the calculation of the first schedule, a weight, $w_{ik}$, of each of the N6 soft constraints can be set to be 1. Hereby, uniform weights for a medical device distribution constraint, MDDC, which can be one of the N6 soft constraints, are set. The weightings of the N6 soft constraints can comprise the weight $w_{ik}$. Furthermore, initially, the allocation error, $e_{ik}$, regarding an allocation of the staff member i to the medical device k, can be set to be 0. Then, by using an allocation matrix, $X_{ij} = \begin{cases} 1 & \text{if staff member i allocated to a shift j} \\ 0 & \text{otherwise} \end{cases}$ , the medical device distribution constraint MDDC can be calculated by MDDC = $\Sigma_{j \in J_k} X_{jj} = R_{ik} + e_{ik}$ , $\forall$ i $\in$ N3, k $\in$ N1 with N1 as the number of medical devices and $J_k$ as a set of shifts belonging to the medical device k. Let the scheduled allocation as generated by the scheduler unit be $S_{ik}$ describing an allocation of the staff member i to the medical device k. Then, the allocation error $e_{ik}$ can be calculated as $e_{ik} = S_{ik} - R_{ik}$. For the calculation of a further planning horizon and/or a further schedule of the N2 schedules, the weight $w_{ik}$ can be adjusted and set to be $w_{ik} = 1 + \alpha \cdot abs(e_{ik})$ with $abs(e_{ik})$ giving the absolute value of the allocation error $e_{ik}$ and $\alpha$ representing a scaling parameter which can be tuned, for example during usage of the system by the scheduler unit and/or by the user. Nonetheless, said example for calculating the allocation error and adjusting the weightings given above is of exemplary manner. Other methods are feasible as well.

Eventually, with the allocation error, a numerical and quantitative value is provided defining the quality of the provided N2 schedules, in particular, of the first schedule with respect to achieving the balanced allocation. In other words, with the allocation error, the scheduler unit and/or the user can evaluate how well the generated first schedule meets the balanced allocation. Possibly in response to the calculated allocation error, the scheduler unit and/or the user can adjust the weightings of the N6 soft constraints, for example, for the calculation of the at least one further schedule of the N2 schedules, followed by a subsequent generation of the at least one further schedule of the N2 schedules. Hereby, the at least one further schedule can cover at least one subsequent planning horizon, for example the respective next week(s) or next month(s).

According to a further embodiment, the scheduler unit is configured to improve the first schedule by: selecting at least one of the N6 soft constraints for improvement by the user; adjusting an allowable violation of the selected at least one of the N6 soft constraints by the user or performing an automatic adjustment of the N6 soft constraints to find a minimum violation of the selected at least one of N6 soft constraints; and updating the first schedule. In particular, the scheduler unit is configured to improve the first schedule by repeating the above-mentioned steps for another one of the N6 soft constraints.

The selected at least one of the N6 soft constraints represents the one or more which shall be improved. For example, the selected at least one soft constraint can be the least weighted one of the N6 soft constraints. Hereby, the improvement can be understood in the sense of reducing possible violations of the selected at least one soft constraint or, which is preferred, of the violations of all of the N6 soft constraints as a whole, that is, an improvement of the overall N2 schedules. The scheduler unit can be configured to keep constant those of the N6 soft constraints and/or respective values addressed with a higher weighting than the selected at least one soft constraint. This way, by adjusting the allowable violation of the selected at least one soft constraint higher rank soft constraints are not altered. The allowable violation can refer to a quantitative and/or qualitative range concerning a given soft constraint in which respective values of the generated N2 schedules have to be located in. A placement of value concerning a given soft constraint outside of said range by the scheduler unit when generating the N2 schedules is considered as a violation of the given soft constraint. For example, the selected at least one soft constraint can represent the working hours per day of a given staff member. The respective allowable violation of that soft constraint may be a range of 6 to 9 working hours per day. This way, when generating the first schedule, the scheduler unit will aim achieve between 6 to 9 working hours for the given staff member for every day, on which the given staff member is working at the medical facility. At the same time, the scheduler unit aims to pay respect to all other hard constraint and soft constraints, possibly causing significant violations of other soft constraints which can lower the overall quality of the generated first schedule. In case the generated first schedule is of poor quality and/or shows many violations of the set of constraints, the user can select the above-mentioned soft constraint representing the working hours per day of the given staff member and, for example, enlarge the range by allowing between 5 to 10 hours per day. A consecutive regeneration of the first schedule can show the effect of said adjustment. In case the generation of the first schedule is not possible with said adjustment, for example, since one or more of the N5 hard constraints cannot be fulfilled anymore, the system can notify the user about that. The adjustment of the selected one of the N6 soft constraints and the consecutive regeneration of the first schedule can be understood as a single optimization step. Alternatively, the system can be configured to perform the automatic adjustment of the N6 soft constraints. There, the scheduler unit can be configured to automatically find a minimum violation of the selected at least one soft constraint by performing one or more optimization steps. The scheduler unit can be configured to perform a binary search method in order to reduce the violation ranges of the N6 soft constraints. Nonetheless, other methods for identifying the minimum violation of the selected at least one soft constraint are feasible, as well. If desired by the user, the above-mentioned optimization steps can be repeated for another one of the N6 soft constraints in order to further improve the first schedule. The above-mentioned optimization steps are described for the generation of the first schedule. Nonetheless, said optimization steps can be applied in order to improve any further one of the N2 schedules, as well. In general, throughout the whole application, everything mentioned regarding the first schedule can be valid for every at least one further schedule, as well. Furthermore, at least one of the above-mentioned steps can be automatically performed by the scheduler unit, as well.

According to a further embodiment, the scheduler unit is configured to respond to the adjustment of the allowable violation by the user by performing a single optimization step while keeping the violation of those of the N6 soft constraints with a higher weighting than the selected at least one of the N6 soft constraints.

The N6 soft constraints with a higher weighting than the selected at least one of the N6 soft constraints can represent, for example, such soft constraints which are considered to be more important than other soft constraints. This way, when performing the single optimization step, the scheduler unit can be configured to adjust the values of such soft constraints with a lower weighting first while the ones with a higher weighting remain constant or are adjusted afterwards. This approach provides a minimal-invasive optimization of the first schedule.

According to a further embodiment, the scheduler unit is configured to perform the automatic adjustment of the N6 soft constraints during multiple optimization steps using a binary search method, in particular, by iteratively reducing violation ranges.

When performing the binary search method, in a first optimization step, the scheduler unit is configured to split an initial violation range of a given soft constraint into two halves. The scheduler unit is configured to identify in which of the halves an optimal solution is located. The identified half becomes a second violation range for a second optimization step. Then, the scheduler unit is configured to split the second violation range again into two halves, and so on. Hereby, the number of optimization steps is arbitrary. The binary search method provides a fast optimization of the first schedule by finding the minimum violations rapidly.

According to a further embodiment, the scheduler unit is configured to provide the N2 schedules such way that each member of the N4 total medical staff is assigned to every one of the N1 medical devices in a balanced manner.

Hereby, a balanced allocation can be achieved. Note that the precision of the balanced allocation can be improved with the number N2. That is, the more schedules are generated, the more precisely the balanced allocation can be achieved. This way, a gain in experience in using every one of the N1 medical devices by every member of the N4 total medical staff can be achieved.

According to a further embodiment, the scheduler unit comprises a schedule database and/or a constraints database.

Previously generated schedules can be stored within the schedule database for reference, comparison, etc. Furthermore, after their generation, the generated N2 schedules can be stored within the schedule database. In a similar way, previous constraints and their weightings, which have been used for generating the previously generated schedules, can be stored within the constraints database for reference, comparison, etc. After their acquisition, the N6 soft constraints and the weightings can be stored within the constraints database, as well. The previously generated schedules and/or the respective previous constraints and weightings can be used for generating the N2 schedules. For example, the weight of a given soft constraint can be calculated according to the number of times the given soft constraint has been selected in the past for updating the violation range concerning the given soft constraint. In addition, the weight of the given soft constraint can be calculated according to the size of the violation range of the given soft constraint. That is, for example, the smaller or narrower the violation range, the higher the weight of the given soft constraint. Nonetheless, the user can explicitly set the weight of a given soft constraint, as well. With said databases, the system is able to learn from previous usages of the system. The system can use previous schedules, constraints, and/or weightings for the generation of the N2 schedules, minimizing the required input data and/or other user interactions and, eventually, utilize gained knowledge in using the N1 medical devices for improving the future usage of the N1 medical devices and saving time and costs.

According to a further embodiment, the system further comprises a local memory and/or a cloud server, wherein the scheduler unit is configured to acquire the set of constraints and/or the weightings from the user interface, the local memory, and/or the cloud server. In particular, the scheduler unit is configured to read and/or to store the schedule database and/or the constraints database from/to the local memory and/or the cloud server.

The local memory can be a hard drive or any other memory device being integrated within the system. The cloud server can be a server or any other memory device being addressed via an intranet connection, internet connection, and/or other connection means. This way, the system can use previous schedules and/or constraints for the generation of the N2 schedules, minimizing the required input data and/or other user interactions and, eventually, utilize gained knowledge in using the N1 medical devices for improving the future usage of them and saving time and costs. This way, the schedule database and/or the constraints database are usable by other systems, as well.

According to a further embodiment, the scheduler unit comprises a solver unit for providing the N2 schedules, wherein the solver unit is configured to apply an optimization algorithm. In particular, the solver unit is configured to apply a constrained quadratic model solver from a D-Wave library. Furthermore, in particular, the solver unit is configured to compute the optimal set of decision variables using a quantum annealing technique.

In addition, or alternatively, other optimization algorithms can be applied by the solver unit in order to provide the N2 schedules, as well. Hereby, the solver unit can be a computer program product and/or a computer.

According to a further embodiment, the N2 schedules span over N2 planning horizons.

For example, the N2 schedules can span over one, two, three, four, five, or more planning horizons.

According to a further embodiment, the solver unit is configured to minimize a gap between a number of N7 required medical staff and the N3 allocated medical staff.

The N7 required medical staff can represent the minimal number of staff members required to operate the N1 medical devices. Hence, the solver unit can be configured to assign a number of staff members to each of the N1 medical devices as close as possible to the respective required number of medical staff member being required to operate the respective medical device.

Hereby, the required number of staff members can be individual for each medical device. Furthermore, the required number of staff members of a given medical device can vary according to the type of the given medical device and/or the planned type of usage of the given medical device.

According to a further embodiment, the scheduler unit is configured for acquiring input data of the N1 medical devices.

The scheduler unit can be configured to receive data and/or instructions from N1 medical device, for example, addressing the previous and/or current usage of the N1 medical devices by the N3 allocated staff. If, for example, the N1 medical devices detect an improper and/or inefficient usage by the N3 allocated staff, the scheduler unit can be informed and/or instructed to vary the allocation of the N4 total medical staff accordingly, for example, in the next schedule and/or planning horizon. The user can be advised and/or informed concerning that via the user interface. This way, the efficiency of the N1 medical devices can be further improved.

According to a further aspect, a method for using a system with a number N1 of medical devices is suggested. Hereby, the above-mentioned system is particularly embodied according to the above described aspect or according to any embodiment of the above described aspect. The method comprises: acquiring input data by a user interface and adding the acquired input data to a set of constraints; providing a number N2 of schedules based on the acquired input data by a scheduler unit, with $N2 \geq 1$; outputting the provided N2 schedules to a user by the user interface; and controlling the N1 medical devices based on the N2 provided schedules.

According to a further aspect, a computer program product is suggested comprising a program code for executing the above-mentioned method for when run on at least one computer.

The computer program product, such as a computer program means, may be embodied as a memory card, USB stick, CD-ROM, DVD or as a file which may be downloaded from a server in a network. For example, such a file may be provided by transferring the file comprising the computer program product from a wireless communication network.

The embodiments and features described with reference to the system and/or the computer program product of the present invention apply mutatis mutandis to the method of the present invention.

Further possible implementations or alternative solutions of the invention also encompass combinations - that are not explicitly mentioned herein - of features described above or below with regard to the embodiments. The person skilled in the art may also add individual or isolated aspects and features to the most basic form of the invention.

Further embodiments, features and advantages of the present invention will become apparent from the subsequent description and dependent claims, taken in conjunction with the accompanying drawings, in which:

Fig. 1 shows a schematic illustration of a system for allocating medical staff to a number N1 of medical devices;

Fig. 2 shows a schematic diagram showing relations between subgroups of the medical staff for using the system according to Fig. 1;

Fig. 3 shows a schematic illustration of a binary search method used by the system according to Fig. 1; and

Fig. 4 shows a schematic block diagram illustrating method steps for using the system according to Fig. 1.

In the Figures, like reference numerals designate like or functionally equivalent elements, unless otherwise indicated.

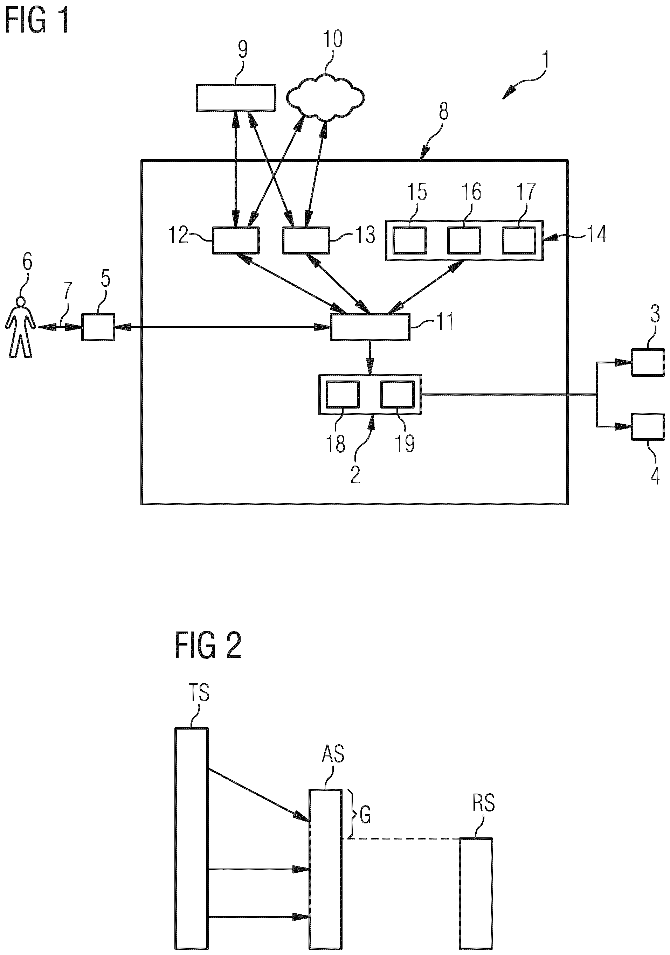

Fig. 1 shows a schematic illustration of a system 1 for allocating medical staff to a number N1 of medical devices 3, 4, with N1 ≥ 1. The system 1 is configured to generate a number N2 of schedules 2, with N2 ≥ 1, by allocating the medical staff to the N1 medical devices 3, 4. That is, to define when and how each of the N1 medical devices 3, 4 is used by which one or more members of the medical staff. This way, the manner of usage of the N1 medical devices 3, 4 is defined.

The system 1 can be located in a medical facility (not shown) in order to allocate the medical staff related to the medical facility to the N1 medical devices 3, 4 related to the medical facility. Hereby, both the number of medical staff members as well as the number N1 of medical devices 3, 4 are arbitrary. In the following, for visualization reasons, only two medical devices 3, 4 are shown.

The system 1 comprises a user interface 5 to enable a user interaction between a user 6 using the system 1 and the system 1. The user interface 5 is configured to receive input data 7 from the user 6 and/or to output data to the user 6.

The system 1 further comprises a scheduler unit 8. The scheduler unit 8 can be implemented as a computer program product or represent a computer (not shown). The scheduler unit 8 is in communication with the user interface 5. Furthermore, the scheduler unit 8 is in communication with a local memory 9 and/or a cloud server 10. The local memory 9 can be a hard drive of the computer (not shown) on which computer the system 1 is implemented. The scheduler unit 8 is configured to transfer data via the Internet and/or to store data on the cloud server 10.

The scheduler unit 8 comprises a solver unit 11 in order to process the input data 7, the data of the local memory 9, and/or the cloud server 10. The solver unit 11 can be configured to apply an optimization algorithm, in particular, a constrained quadratic model solver from a D-Wave library. For example, the solver unit 11 can be configured to allocate the medical staff members to the N1 medical devices 3, 4 by computing an optimal set of decision variables using a quantum annealing technique.

The scheduler unit 8 comprises a constraints database 12 on which previous constraints (not shown) and/or previous weightings (not shown) from previous usages of the system 1 can be stored. Constraints being part of the input data 7 can be stored in the constraints database 12 as well. In addition, the scheduler unit 8 comprises a schedule database 13 for storing schedules previously generated by the scheduler unit 8.

The scheduler unit 8 further comprises a set of constraints 14. The set of constraints 14 comprises all constraints relevant for generating the N2 schedules 2. That is, all factors defining degree of freedoms inside which the solver unit 11 can produce solutions for the N2 schedules 2. The scheduler unit 8 is configured to save all constraints received via the input data 7 as well as relevant constraints from the constraints database 12 within the set of constraints 14. Hereby, the constraints are subdivided according to their importance. Eventually, the set of constraints 14 comprises a number N5 of hard constraints 15, with N5 ≥ 1, which are mandatory to fulfill when generating the N2 schedules 2, as well as a number N6 of soft constraints 16, with N6 ≥ 1, which are optional but desired to fulfill. In addition, the set of constraints 14 comprises weightings 17 which define the importance of each of the N6 soft constraints 16 in relation to the respective other N6 soft constraints 16 as well as the relevance of the N6 soft constraints 16 as a whole in relation to the N5 hard constraints 15. The scheduler unit 8 is configured to generate the N2 schedules 2 based on the set of constraints 14. The N2 schedules 2 comprise a first schedule 18 which is generated first, as well as optionally, at least one further schedule 19 which is generated after the first schedule 18. Hence, the existence of the at least one further schedule 19 is of exemplary manner. The system 1 can be used to generate solely the first schedule 18, as well. The number of further schedules 19 is arbitrary. Each of the first schedule 18 and the at least one further schedule 19 can cover one planning horizon, for example one week or one month.

Fig. 2 shows a schematic diagram showing relations between subgroups of the medical staff for using the system 1.

The medical staff of the medical facility can be subdivided into a number N4 of total medical staff TS, N4 ≥ 1, comprising all medical staff members related to the medical facility. When generating the N2 schedules 2, the solver unit 11 is configured to select a number N3 of allocated medical staff AS, with N3 ≥ 1. The N3 allocated medical staff AS represent those members of the N4 total medical staff TS which are scheduled to work with the N1 medical devices 3, 4 in the frame of the generated N2 schedules 2. In other words, possibly, not every member of the N4 total medical staff TS may be allocated to work with one of the N1 medical devices 3, 4 within the planning horizons covered by the N2 schedules 2. Therefore, the number N3 is similar or smaller than the number N4. In Fig. 2, said selection process performed by the solver unit 11 is illustrated by three arrows.

The medical staff can further comprise a subgroup with a number N7 of required medical staff RS, N7 ≥ 1, representing an ideal number of medical staff members which are required in order to operate the N1 medical devices 3, 4. When selecting the N3 allocated medical staff AS, the solver unit 11 is configured to allocate at least the N7 required medical staff RS. That is, to provide the N1 medical devices 3, 4 with at least as many medical staff members as required in order to operate the N1 medical devices 3, 4. On the other hand, in case the solver unit 11 allocates more medical staff members to the N1 medical devices 3, 4 as required, a gap G is formed, representing an overrepresentation of the N3 allocated medical staff AS. For economic and efficiency reasons, the solver unit 11 can be configured to minimize the gap G when generating the N2 schedules 2.

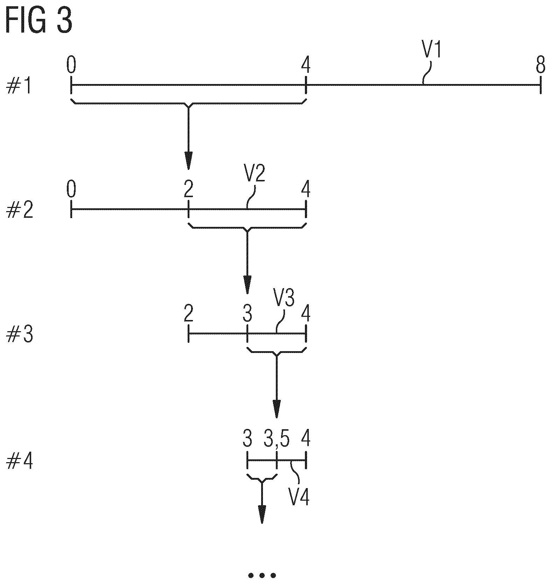

Fig. 3 shows a schematic illustration of a binary search method used by the system 1.

When generating the N2 schedules 2, the solver unit 11 is configured to allocate the medical staff members to the N1 medical devices 3, 4 while fulfilling all of the N5 hard constraints 15 as well as fulfilling the N6 soft constraints 16 as much as possible. Possible violations of the N6 soft constraints 16 are allowed but shall be avoided. In order to minimize the violations of the N6 soft constraints 16, the solver unit 11 can be configured to automatically identify an optimum regarding a given soft constraint 16 using the binary search method. Hereby, in a first step #1, an initial violation range V1 of the given soft constraint 16 is split into two halves. The solver unit 11 is configured to identify inside which of the two halves of the initial violation range V1 the optimal solution is located, representing a second violation range V2 for a second step #2. Then, the second violation range V2 is split again into two halves, and so on. Hereby, the number of steps #1, #2, #3, etc. is arbitrary.

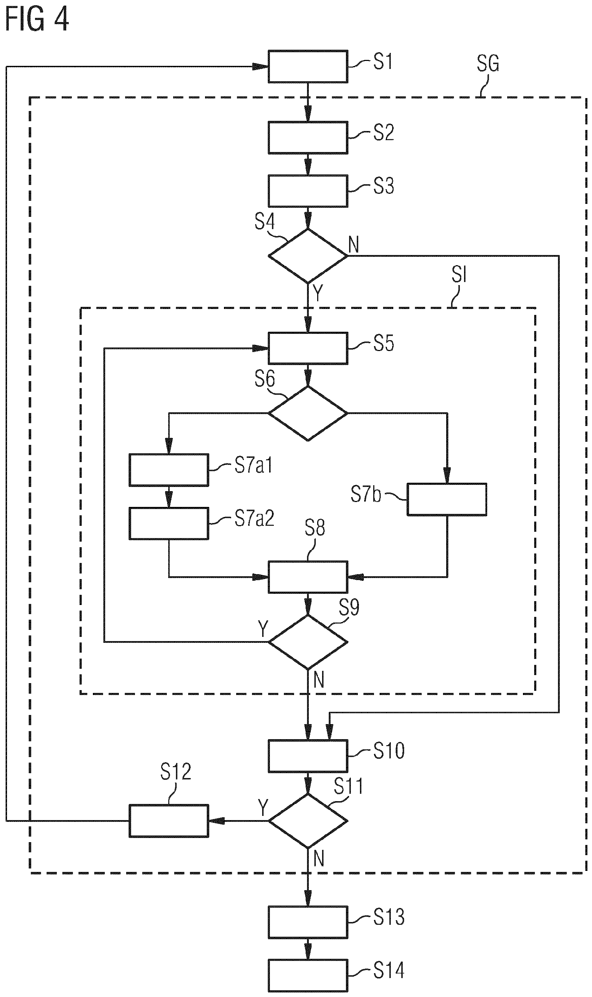

Fig. 4 shows a schematic block diagram illustrating method steps for using the system 1.

In a first step S1, the input data 7 is acquired by the user interface 5. From the user interface 5, the input data 7 can be read by the solver unit 11. Furthermore, the solver unit 11 can read previous constraints and/or previous weightings from the constraints database 12 as well as previous schedules from the schedule database 13. With said data, the solver unit 11 forms the set of constraints 14 with the N5 hard constraints 15, the N6 soft constraints 16, and the weightings 17. In a further step SG, the solver unit 11 generates the N2 schedules 2. Hereby, the step SG comprises a number of sub steps which are explained in detail in the following.

In step S2, the first schedule 18 is generated. Hereby, the first schedule 18 can cover one planning horizon, for example one week or one month. The solver unit 11 aims to fulfill all N5 hard constraints 15 as well as aims to fulfill the N6 soft constraints 16 as precisely as possible. Hereby, the solver unit 11 respects the individual weightings 17 of the N6 soft constraints 16. For generating the first schedule 18, the solver unit 11 applies an optimization algorithm, in particular, a constrained quadratic model solver from a D-Wave library (not shown). In particular, the solver unit 11 computes the optimal set of decision variables using a quantum annealing technique. The generated first schedule 18 is displayed to the user 6 together the possible violations of the N6 soft constraints 16 which occur according to the generated first schedule 18. Hereby, the user 6 is assisted in deciding whether the generated first schedule 18 is appropriate or requires further improvement. In a further step S4, the user 6 can instruct the system 1 via the user interface 5 whether a respective improvement of the generated first schedule 18 shall be performed or not. Hereby, improving can be understood as modifying the generated first schedule 18 such way that the generated first schedule 18 meets the set of constraints 14 with fever violations and/or with a lower degree of violation of the set of constraints 14. In case no further improvement is required, the method continues in step S10, which will be explained in detail below. Otherwise, the first schedule 18 is improved in step SI.

Hereby, the step SI comprises a number of sub steps which will be explained in detail in the following.

In step S5, the user interface 5 enables a user selection of one of the N6 soft constraints 16 which shall be improved in order to adjust the first schedule 18. In a further step S6, the user can further decide whether the selected soft constraint 16 shall be improved by using a single run improvement (see steps S7a1 and S7a2 which will be explained in detail in the following) or by using an automatic multi-run improvement (see step S7b which will be explained in detail below).

In case the user 6 decides for an improvement by using the single run improvement, in step S7a1, the user interface 5 allows an adjustment of the allowable violation range (V1, V2, V3, V4) of the selected soft constraint 16. Here, the user 6 can adjust which values of the first schedule 18 concerning the selected soft constraint 16 are defined as allowable. Hereby, for example, text inputs and/or inputs via a graphical user interface including buttons (not shown) are feasible. In step S7a2, the solver unit 11 performs a single improvement step, that is, a recalculation of the other values concerning the N6 soft constraints 16 and/or the N5 hard constraints 15 by the solver unit 11. Alternatively, in case the user 6 decides for an improvement by using the automatic multi-run improvement, in step S7b, the solver unit 11 performs multiple optimization steps for improving the violation of the selected soft constraint 16 by adjusting all other soft constraints 16 in order to identify an optimal solution for the first schedule 18. Hereby, the solver unit 11 applies the binary search method as illustrated schematically with reference to Fig. 3.

After performing the steps S7a1 and S7a2, or S7b, the method continues in a further step S8, where in the first schedule 18 is updated, respecting possible adjustments due to the performed optimization explained above. In a further step S9, the user 6 can influence via the user interface 5 whether a further soft constraint 16 shall be optimized or not. In case another soft constraint 16 shall be optimized, steps S5 to S8 are repeated for the further soft constraint 16. Otherwise, the method continues with step S10.

In step S10, an allocation error is calculated for the first schedule 18. Possible violations of the N6 soft constraints 16 can cause deviations between a scheduled allocation of the first schedule 18 and a required allocation. Hereby, the scheduled allocation represents the allocation of the N3 allocated medical staff AS to the N1 medical devices 3, 4 as calculated by the solver unit 11 for the generated first schedule 18. On the other hand, the required allocation represents such an allocation which allows for a balanced allocation to be achieved. The balanced allocation represents the ideal condition where every one of the N4 total medical staff TS has the same level of experience concerning the usage of a specific one of the N1 medical devices 3, 4. In other words, the allocation of the N4 total medical staff TS changes over time such way that every one of the N4 total medical staff TS gains experience in using every one of the N1 medical devices 3, 4. In addition, or alternatively, the balanced allocation can be understood as such allocation, where staff members with higher levels of experience are assigned to work with staff members with lower levels of experience in order to provide a transfer of experience. Possibly, with the calculated first schedule 18, the balance allocation is not met precisely. Then, the allocation error describes a degree of a possible deviation of the selected values for each of the N6 soft constraints 16 concerning their respective violation ranges V1, V2, V3, V4. The calculated allocation error can be shown to the user 6 via the user interface 5.

In a further step S11, the user 6 can decide by using the user interface 5 whether at least one further schedule 19 shall be generated. If so, the weightings 17 can be adjusted by the solver unit 11 according to the calculated allocation error. Hereby, specific soft constraints 16 enabling a convergence towards the balanced allocation can be matched with a higher weighting 17. Then, the steps S1 to S11 are repeated in order to generate the at least one further schedule 19. The generation of the at least one further schedule 19 follows the instructions for the first schedule 18. Hence, all explanations concerning the first schedule 18 are relevant for the at least one further schedule 19, as well. The number of further schedules 19 is arbitrary. If no further schedule 19 shall be generated, the procedure follows in step S13 which will be explained in the following. Eventually, the generated first schedule 18 and each further schedule 19 form the N2 schedules 2.

In a further step S13, the generated N2 schedules 2 are output to the user 6 using the user interface 5. For example, the user interface 5 can display the N2 schedules 2 to the user 6 and/or print the N2 schedules 2. In addition, the N2 schedules 2 are stored within the schedule database 13 and the set of constraints 14 is saved in the constraints database 12.

Finally, in step S14, the N1 medical devices 3, 4 are controlled based on the generated N2 schedules 2. That is the manner how, by whom of the N4 total medical staff TS, and in which way the N1 medical devices 3, 4 are used in the frame of the one or more planning horizons covered by the N2 schedules 2 as instructed by the N2 schedules 2. For example, the N2 schedules 2 can comprise information which operation of a given one of the N1 medical devices 3, 4 is planned for which time and/or in which order. Hence, the usage of the N1 medical devices 3, 4 is controlled by the N2 schedules 2.

Although the present invention has been described in accordance with preferred embodiments, it is obvious for the person skilled in the art that modifications are possible in all embodiments.

List of Reference

| | |
|---|---|
| 1 | system |
| 2 | schedules |
| 3 | medical device |
| 4 | medical device |
| 5 | user interface |
| 6 | user |
| 7 | input data |
| 8 | scheduler unit |
| 9 | local memory |
| 10 | cloud server |
| 11 | solver unit |
| 12 | constraints database |
| 13 | schedule database |

EP 4 765 153 A1

| 14 | set of constraints |
| 15 | hard constraint |
| 16 | soft constraint |
| 17 | weightings |
| 18 | first schedule |
| 19 | further schedule |
| | |
| AS | allocated medical staff |
| G | gap |
| i | staff member |
| j | shift |
| J | set of shifts |
| k | medical device |
| M1 | medical device |
| M2 | medical device |
| M3 | medical device |
| M4 | medical device |
| M5 | medical device |
| MDDC | medical device distribution constraint |
| N1 | number of medical devices |
| N2 | number of schedules |
| N3 | number of allocated medical staff |
| N4 | number of total medical staff |
| N5 | hard constraints |
| N6 | soft constraints |
| $R_{ik}$ | allocation value |
| RS | required medical staff |
| S1 | step |
| S2 | step |
| S3 | step |
| S4 | step |
| S5 | step |
| S6 | step |
| S7a1 | step |
| S7a2 | step |
| S7b | step |
| S8 | step |
| S9 | step |
| S10 | step |
| S11 | step |
| S12 | step |
| S13 | step |
| S14 | step |
| SG | step |
| SI | step |
| t1 | number of staff |
| t2 | number of staff |
| t3 | number of staff |
| t4 | number of staff |
| t5 | number of staff |
| TS | total medical staff |
| V1 | violation range |
| V2 | violation range |
| V3 | violation range |
| V4 | violation range |
| $w_{ik}$ | weight |
| X | allocation matrix |

11

**Claims**

1. A system (1) for allocating medical staff to a number N1 of medical devices (3, 4), with N1 ≥ 1, and for controlling the N1 medical devices (3, 4), the system (1) comprising:

   a user interface (5) for acquiring input data (7) and adding the acquired input data (7) to a set of constraints (14);
   a scheduler unit (8) for providing a number N2 of schedules (2) by allocating a number N3 of allocated medical staff (AS) out of a number N4 of total medical staff (TS) to the N1 medical devices (3, 4) and/or to a working shift based on the set of constraints (14), with N2 ≥ 1, N3 ≥ 1, and N4 ≥ 1; and
   the N1 medical devices (3, 4);
   wherein the system (1) is configured to control the N1 medical devices (3, 4) based on the N2 provided schedules (2).

2. The system according to claim 1, wherein the set of constraints (14) comprises a number N5 of hard constraints (15), a number N6 of soft constraints (16), and/or weightings (17) of the N6 soft constraints (16), with N5 ≥ 1 and N6 ≥ 1.

3. The system according to claim 2, wherein the N6 soft constraints (16) comprise a required allocation of the N4 total medical staff (TS) to the N1 medical devices (3, 4), in particular, the weightings (17) comprise a weighting of the required allocation in relation to the others of the N6 soft constraints (16).

4. The system according to claim 3, wherein the scheduler unit (8) is configured to provide the N2 schedules (2) by:

   S2) generating a first schedule (18) of the N2 schedules (2) based on the set of constraints (14), in particular by setting a predefined initial distribution for a machine distribution constraint, wherein the machine distribution constraint is one of the N6 soft constraints (16);
   S3) displaying the generated first schedule (18) to a user (6) of the system (1);
   SI) optionally improving the generated first schedule (18); and
   S10) calculating an allocation error of each of the N6 soft constraints based on a deviation between a scheduled allocation of the first schedule (18) and the required allocation;
   S12) in particular, the scheduler unit (8) is configured to provide the N2 schedules (2) by adjusting the weightings (17) for at least one further schedule (19) of the N2 schedules (2) and repeating steps S2 to S10 for the at least one further schedule (19).

5. The system according to claim 4, wherein the scheduler unit (8) is configured to improve (SI) the first schedule (18) by:

   S5) selecting at least one of the N6 soft constraints (16) for improvement by the user (6);
   S7a) adjusting an allowable violation of the selected at least one of the N6 soft constraints (16) by the user (6) or
   S7b) performing an automatic adjustment of the N6 soft constraints (16) to find a minimum violation of the selected at least one of N6 soft constraints (16); and
   S8) updating the first schedule (18);
   in particular, the scheduler unit (8) is configured to improve (SI) the first schedule (18) by repeating the steps S5 to S8 for another one of the N6 soft constraints (16).

6. The system according to claim 5, wherein the scheduler unit (8) is configured to respond to the adjustment (S7a) of the allowable violation by the user (6) by performing (S7a2) a single optimization step while keeping the violation of those of the N6 soft constraints (16) with a higher weighting than the selected at least one of the N6 soft constraints (16).

7. The system according to claim 5 or 6, wherein the scheduler unit (8) is configured to perform (S7b) the automatic adjustment of the N6 soft constraints (16) during multiple optimization steps using a binary search method, in particular, by iteratively reducing violation ranges (V1 - V4).

8. The system according to one of claims 1 to 7, wherein the scheduler unit (8) is configured to provide the N2 schedules (2) such way that each member of the N4 total medical staff (TS) is assigned to every one of the N1 medical devices (3, 4) in a balanced manner.

9. The system according to one of claims 1 to 8, wherein the scheduler unit (8) comprises a schedule database (13) and/or a constraints database (12).

10. The system according to claim 9, further comprising a local memory (9) and/or a cloud server (10), wherein the scheduler unit (8) is configured to acquire the set of constraints (14) and/or the weightings (17) from the user interface (5), the local memory (9), and/or the cloud server (10), in particular, the scheduler unit (8) is configured to read and/or to store the schedule database (13) and/or the constraints database (12) from/to the local memory (9) and/or the cloud server (10).

11. The system according to one of claims 1 to 10, wherein the scheduler unit (8) comprises a solver unit (11) for providing the N2 schedules (2), wherein the solver unit (11) is configured to apply an optimization algorithm, in particular, a constrained quadratic model solver from a D-Wave library, in particular, the solver unit (11) is configured to compute an optimal set of decision variables using a quantum annealing technique.

12. The system according to one of claims 1 to 11, wherein the N2 schedules (2) span over N2 planning horizons.

13. The system according to claims 11 or 12, wherein the solver unit (11) is configured to minimize a gap (G) between a number of N7 required medical staff (RS) and the N3 allocated medical staff (AS).

14. The system according to one of claims 1 to 13, wherein the scheduler unit (8) is configured for acquiring input data (7) of the N1 medical devices (3, 4).

15. A method for using a system (1) with a number N1 of medical devices (3, 4), the system (1) particularly being embodied according to one of claims 1 to 13, the method comprising:

   S1) acquiring input data (7) by a user interface (5) and adding the acquired input data (7) to a set of constraints (14);
   SG) providing a number N2 of schedules (2) based on the acquired input data (7) by a scheduler unit (8), with N2 $\geq$ 1;
   S13) outputting the provided N2 schedules (2) to a user (6) by the user interface (5); and
   S14) controlling the N1 medical devices (3, 4) based on the N2 provided schedules (2).

FIG 1

FIG 2

# FIG 3

# FIG 4

Europäisches
Patentamt
European
Patent Office
Office européen
des brevets

# EUROPEAN SEARCH REPORT

**Application Number**

EP 24 22 2782

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/039906 A1 (WANG HAIYAN [US] ET AL) 6 February 2014 (2014-02-06) * The whole document, in particular: Paragraphs: [0009] - [0015], [0022], [0026], [0027], [0031], [0037], [0045]. Figures: Fig. 1, Fig. 2. * | 1-15 | INV. G16H40/20 |
| A | US 2023/018946 A1 (USHIJIMA-MWESIGWA HAYATO [US] ET AL) 19 January 2023 (2023-01-19) * The whole document, in particular: Paragraphs: [0036] - [0039]. * | 1-15 | |
| A | EP 3 136 308 A1 (SERVICEPOWER BUSINESS SOLUTIONS LTD [GB]) 1 March 2017 (2017-03-01) * The whole document, in particular: Paragraphs: [0007], [0014] - [0016]. * | 1-15 | |
| A | US 2017/060623 A1 (SYRICHAS ALEX [GB] ET AL) 2 March 2017 (2017-03-02) * The whole document, in particular: Paragraphs: [0002] - [0041]. * | 1-15 | TECHNICAL FIELDS SEARCHED (IPC) G16H |
| A | TSAO YU-CHUNG ET AL: "Intelligent Clinic Nurse Scheduling Considering Nurses Paired with Doctors and Preference of Nurses", JOURNAL OF MEDICAL SYSTEMS, SPRINGER US, NEW YORK, vol. 48, no. 1, 12 August 2024 (2024-08-12), XP038034415, DOI: 10.1007/S10916-024-02092-W [retrieved on 2024-08-12] * The whole document, in particular: Page 75, Col. 2. * | 1-15 | |

The present search report has been drawn up for all claims

| Place of search | Date of completion of the search | Examiner |
|---|---|---|
| Munich | 9 May 2025 | Zacharias, Malte |

EPO FORM 1503 03.82 (P4C01)

## ANNEX TO THE EUROPEAN SEARCH REPORT
## ON EUROPEAN PATENT APPLICATION NO.

EP 24 22 2782

This annex lists the patent family members relating to the patent documents cited in the above-mentioned European search report.
The members are as contained in the European Patent Office EDP file on
The European Patent Office is in no way liable for these particulars which are merely given for the purpose of information.

09-05-2025

| Patent document cited in search report | Publication date | Patent family member(s) | | Publication date |
|---|---|---|---|---|
| US 2014039906 A1 | 06-02-2014 | NONE | | |
| US 2023018946 A1 | 19-01-2023 | EP | 4113402 A1 | 04-01-2023 |
| | | JP | 2023007459 A | 18-01-2023 |
| | | US | 2023018946 A1 | 19-01-2023 |
| EP 3136308 A1 | 01-03-2017 | NONE | | |
| US 2017060623 A1 | 02-03-2017 | EP | 3136312 A1 | 01-03-2017 |
| | | US | 2017060623 A1 | 02-03-2017 |

EPO FORM P0459

For more details about this annex : see Official Journal of the European Patent Office, No. 12/82